(19) Europäisches Patentamt

European Patent Office

Office européen des brevets

(11) Veröffentlichungsnummer: **0 254 803**
**A2**

(12) # EUROPÄISCHE PATENTANMELDUNG

(21) Anmeldenummer: 87104634.8

(22) Anmeldetag: 27.03.87

(51) Int. Cl.⁴: **C07D 295/22**

(30) Priorität: 31.05.86 DE 3618352

(43) Veröffentlichungstag der Anmeldung:
**03.02.88 Patentblatt 88/05**

(84) Benannte Vertragsstaaten:
**AT BE CH DE ES FR GB IT LI NL SE**

(71) Anmelder: **HÜLS AKTIENGESELLSCHAFT**
**- RSP Patente / PB 15 - Postfach 13 20**
**D-4370 Marl 1(DE)**

(72) Erfinder: **Scholten, Heinz, Dr.**
**Rehwinkel 3**
**D-4358 Haltern 7(DE)**
Erfinder: **Rindtorff, Klaus, Dr.**
**Keplerstrasse 13**
**D-4350 Recklinghausen(DE)**

(54) **Verfahren zur Herstellung von wässrigen N-Methylmorpholin-N-oxid-Lösungen.**

(57) Zur Herstellung von N-Methylmorpholin-N-oxid setzt man das Methylmorpholin-Wasser-Azeotrop mit einer wäßrigen Wasserstoffperoxidlösung um und konzentriert die Reaktionslösung anschließend auf den gewünschten Gehalt an N-Methylmorpholin-N-oxid auf.

EP 0 254 803 A2

## Verfahren zur Herstellung von wäßrigen N-Methylmorpholin-N-oxid-Lösungen

N-Methylmorpholin-N-oxid ist nach der DE-PS 16 94 048 ein gutes Lösemittel für Cellulose und eignet sich deshalb hervorragend für die Faserherstellung.

Die mit seiner Hilfe hergestellten Cellulosefasern zeichnen sich gegenüber den herkömmlichen durch eine verbesserte Reißfestigkeit aus.

Weitere Einsatzgebiete sind Zwischenprodukte für Pharmazeutika. Da N-Methylmorpholin-N-oxid nur eine begrenzte Lagerstabilität aufweist und als reiner Stoff zur Sauerstoffabspaltung neigt, ist seine bevorzugte Handelsform die 60 %ige wäßrige Lösung, in der es auch zur Anwendung kommt.

Wichtig für Lagerstabilität und Anwendungen sind jedoch eine hohe Reinheit der Lösung. Übliche nachgewiesene Verunreinigungen, die die Lagerstabilität herabsetzen oder bei der Anwendung stören, sind N-Methylmorpholin, Peroxide und saure Komponenten. Die Verunreinigungen machen sich im Handelsprodukt in einer intensiven Gelbfärbung bemerkbar.

Die Verunreinigungen sind analytisch erfaßbar. So sind beispielsweise bei der potentiometrischen Titration an handelsüblichem N-Methylmorpholin-N-oxid mit Salzsäure und Natronlauge zwei Potentialsprünge zu beobachten, die freiem Methylmorpholin und Carboxylgruppen zuzuordnen sind. Weiterhin sind durch jodometrische Titration Peroxide bestimmbar, die, da sie nicht exakt identifiziert werden können, als Wasserstoffperoxid berechnet werden.

Handelsübliches N-Methylmorpholin-N-oxid enthält bis zu 1 Gew.-% Methylmorpholin, bis zu 0,01 Gew.-% Peroxide, gerechnet als Wasserstoffperoxid, und bis zu 0,2 Gew.-% Carboxylgruppen.

Werden nach dem Stand der Technik annähernd äquimolare Mengen Wasserstoffperoxid (35 %ig) und handelsübliches Methylmorpholin (99 %ig) zur Reaktion gebracht, so entsteht eine 57,4 %ige Methylmorpholin-N-oxid-Lösung, die als Verunreinigungen 0,3 Gew.-% Peroxid (gerechnet $H_2O_2$), 0,97 Gew.-% Methylmorpholin und 0,3 Gew.-% Carboxylgruppen aufweist. Da nach dem Aufkonzentrieren auf die handelsübliche Konzentration von 60 Gew.-% Methylmorpholin-N-oxid die Konzentration der Verunreinigungen noch zunimmt, entspricht das Produkt nicht dem Stand der Technik. Da vor allem der Peroxidgehalt zu hoch ist, wird in der Literatur vorgeschlagen, diesen durch Zugabe von 0,1 Gew.-% Katalase-Enzym zu zersetzen. Die Katalase bedeutet jedoch eine weitere Verunreinigung, so daß eine aufwendige Reinigung angeschlossen werden muß, die folgende Schritte umfaßt:

1. Entfernung des Wassers durch Azeotropdestillation mit Benzol.
2. Trennung der Methylmorpholin-N-oxid-Phase von Benzol.
3. Fällung des Methylmorpholin-N-oxids mit Aceton.
4. Filtration und Vakuumtrocknung des Methylmorpholin-N-oxids.
5. Herstellung der Lösung.

Dieses Verfahren ist sehr aufwendig.

Hieraus ergab sich die Aufgabe, ein Verfahren zu finden, das in einfacher und wirtschaftlicher Weise die Herstellung von reinen, wäßrigen Lösungen von N-Methylmorpholin-N-oxid-Lösungen ermöglicht.

Diese Aufgabe wird erfindungsgemäß entsprechend den Angaben der Patentansprüche gelöst.

Überraschenderweise wurde gefunden, daß man eine hochreine N-Methylmorpholin-N-oxid-Lösung erhält, wenn man das Einsatzprodukt Methylmorpholin zunächst mit Wasser versetzt, diese Mischung destilliert, das erhaltene Kopfprodukt, das Azeotrop, mit Wasserstoffperoxid umsetzt und das entstandene Reaktionsprodukt durch teilweise Abdestillation des Wassers aufkonzentriert, bevorzugt auf eine Konzentration von ca. 60 Gewichtsprozent.

Die Mischungen Methylmorpholin und Wasser enthalten bevorzugt 50 bis 75 % Methylmorpholin und 25 bis 50 % Wasser. Insbesondere setzt man Mischungen von 50 % Methylmorpholin und 50 % Wasser ein. Methylmorpholin und Wasserstoffperoxid setzt man bevorzugt im Molverhältnis von 1 : 0,9 bis 1 : 0,75 ein. Die Umsetzung von Methylmorpholin mit Wasserstoffperoxid erfolgt bei Temperaturen von 60 bis 100 °C, vorzugsweise bei 70 bis 75 °C.

Die Reaktionszeit beträgt im allgemeinen 4 bis 6 Stunden. Die Nachreaktion erfolgt im allgemeinen bei der gleichen Temperatur. Das eingesetzte Wasserstoffperoxid hat im allgemeinen eine Konzentration von 30 bis 65 %, bevorzugt 30 bis 35 %.

Das Verfahren läßt sich auch kontinuierlich betreiben, wobei die Konzentration des Endproduktes, vorzugsweise ca. 60 Gewichtsprozent, mit Hilfe des Brechungsindex oder einer Dichtemessung überwacht werden kann. Die so hergestellte N-Methylmorpholin-N-oxid-Lösung ist nur leicht gelblich gefärbt und enthält als Verunreinigungen kaum noch nachzuweisende Mengen Peroxid, Methylmorpholin und Carboxylgruppen. Sie ist für alle bekannten Anwendungsfälle direkt einsetzbar.

### Beispiel

600 g Methylmorpholin und 600 g Wasser werden mit Rücklaufverhältnis 1 über eine 2-m-Füllkörperkolonne destilliert. Am Kopf werden 705 g Azeotrop abgenommen. Das Produkt enthält 74,5 % Methylmorpholin.

680 g des Methylmorpholin-Azeotrops werden unter Rühren bei 70 °C innerhalb 2 Stunden mit 400 g Wasserstoffperoxid (35 %ig) umgesetzt und anschließend weitere 6 Stunden bei 68 °C weitergerührt. Dann wird im Vakuum auf 803 g aufkonzentriert. Erhalten wird folgende wäßrige N-Methylmorpholin-N-oxid-Lösung:

Gehalt an N-Methylmorpholin-N-oxid 59,4 %
Peroxid als $H_2O_2$ gerechnet 2 ppm
Methylmorpholin 0,1 %
Säurezahl 0,1 mg KOH/g

Das abdestillierte Wasser enthält 34 Gew.-% Methylmorpholin. Es wird nach destillativer Aufkonzentrierung wieder eingesetzt.

### Vergleichsbeispiel nach der DE-PS 16 94 048

(Das Beispiel wurde insofern abgeändert, als auf die Zersetzung des überschüssigen Peroxids verzichtet und wie im Beispiel aufkonzentiert wurde.)

In einem Dreihalskolben werden 650 g N-Methylmorpholin und 50 g Wasser auf 68 °C erhitzt und anschließend innerhalb von 2 Stunden bei 70 °C 485 g einer 35 %igen Wasserstoffperoxidlösung zugegeben. Nach beendeter Zugabe wird die Reaktionsmischung noch 2 Stunden auf einer Temperatur von 68 bis 70 °C gehalten und anschließend bei 70 °C im Vakuum auf 974 g eingedickt. Es wird folgende intensiv gelb bis braun gefärbte Lösung erhalten:

Gehalt an N-Methylmorpholin-N-oxid 59 %
Peroxid als $H_2O_2$ gerechnet 0,21 %
Methylmorpholin 0,24 %
Säurezahl 5,3 mg KOH/g

### Ansprüche

1. Verfahren zur Herstellung einer wäßrigen, reinen N-Methylmorpholin-N-oxid-Lösung durch Umsetzung von Methylmorpholin mit einer wäßrigen Wasserstoffperoxidlösung,
dadurch gekennzeichnet,
daß man Mischungen von Methylmorpholin mit Wasser destilliert, das Methylmorpholin-Wasser-Azeotrop bei Temperaturen von 60 bis 100 °C mit einer wäßrigen Wasserstoffperoxidlösung umsetzt und die Reaktionslösung anschließend auf den gewünschten Gehalt an N-Methylmorpholin-N-oxid aufkonzentriert.

2. Verfahren nach Anspruch 1,
dadurch gekennzeichnet,
daß man Methylmorpholin und Wasserstoffperoxid im Molverhältnis 1 : 0,9 bis 1 : 0,75 einsetzt.

3. Verfahren nach Anspruch 1 und 2,
dadurch gekennzeichnet,
daß man die Umsetzung bei einer Temperatur von 70 bis 75 °C durchführt.

4. Verfahren nach einem der Ansprüche 1 bis 3,
dadurch gekennzeichnet,
daß man eine 30 bis 65 %ige wäßrige Wasserstoffperoxidlösung einsetzt.

5. Verfahren nach einem der Ansprüche 1 bis 4,
dadurch gekennzeichnet,
daß man Mischungen von 50 bis 75 % Methylmorpholin und 25 bis 50 % Wasser, vorzugsweise eine Mischung von 50 % Methylmorpholin und 50 % Wasser, destilliert.